# EUROPÄISCHE PATENTSCHRIFT

(11) **EP 1 110 932 B1**
(45) Veröffentlichungstag und Bekanntmachung des Hinweises auf die Patenterteilung: **12.04.2006**
(21) Anmeldenummer: 00126904.2
(22) Anmeldetag: 08.12.2000
(51) Int. Cl.: C07C 5/09, C07C 29/17

(54) **Verfahren zur Herstellung von C10-C30-Alkenen durch partielle Hydrierung von Alkinen an Festbett-Palladium-Trägerkatalysatoren**
Process for the preparation of C10-C30-alkenes by partial hydrogenation of alkynes on fixed-bed palladium-supported catalysts
Procédé de préparation d'alcènes en C10-C30 par hydrogénation partielle d'alcynes sur des catalyseurs à lit fixe contenant du palladium

(30) Priorität: 23.12.1999 DE 19962907
(43) Veröffentlichungstag der Anmeldung: 27.06.2001
(73) Patentinhaber: BASF Aktiengesellschaft, 67056 Ludwigshafen (DE)
(72) Erfinder: Reimer, Klaus, 67112 Mutterstadt (DE); Kaibel, Gerd, Dr., 68623 Lampertheim (DE); Kammel, Ulrich, Dr., 67346 Speyer (DE); Bröcker, Franz-Josef, Dr., 67061 Ludwigshafen (DE); Ansmann, Andreas, Dr., 69168 Wiesloch (DE); Etzrodt, Heinz, Dr., 67434 Neustadt (DE); Stroezel, Manfred, 68549 Ilvesheim (DE); Haake, Mathias, Dr., 68161 Mannheim (DE); Laupichler, Lothar, Dr., 67227 Frankenthal (DE); Bockstiegel, Bernhard, Dr., 67354 Römerberg (DE)

(56) Entgegenhaltungen:
- US-A- 4 001 344
- US-A- 4 490 481
- US-A- 4 517 395
- US-A- 5 516 851
- US-A- 5 750 806
- US-A- 5 866 734

## Beschreibung

Die Erfindung betrifft ein sehr vorteilhaftes Verfahren zur technischen Herstellung von höhermolekularen Alkenen, insbesondere von monosubstituierten Alkenen, durch Partialhydrierung der entsprechenden Alkine in flüssiger Phase an Palladium-Festbett-Trägerkatalysatoren unter Zusatz von Kohlenmonoxid (CO) zum Hydrierwasserstoff.

Die Hydrierung von Alkinen zu Alkenen ist technisch von großer Bedeutung und ist daher Gegenstand eines umfangreichen Standes der Technik.

So wird in GB A 871 804 eine verbesserte partielle Hydrierung von Acetylenverbindungen in Suspensionsfahrweise mit einem PalladiumKatalysator (Pd-Katalysator), der mit Salzlösungen der Metalle Zn, Cd, Hg, Ga, In oder Tl dotiert wurde, beschrieben.

Ferner wird in DE A 24 31 929 ein Verfahren zur Herstellung von 2-Buten-1,4-diol durch Hydrieren von Butindiol in wäßriger Lösung an einem Katalysator, der Pd und eines der Elemente Zn oder Cd und wenigstens eines der Elemente Bi oder Te enthält, beschrieben. Als Katalysatorträger wird Bimsstein oder Aluminiumoxid verwendet.

Für die partielle Hydrierung der Dreifachbindung in Vorprodukten für Vitamine und Riechstoffe werden üblicherweise Blei-dotierte Pd-Katalysatoren, sogenannte Lindlar-Katalysatoren eingesetzt (vgl. beispielsweise in US 2 681 938).

Vielfach werden diese Lindlar-Katalysatoren noch mittels Schwefelverbindungen desaktiviert, um die Selektivität zu steigern (vgl. JP A 120 657/81).

In US 2 809 215 wird die diskontinuierliche Hydrierung von 3,7,11-Trimethyl-6-dodecen-1-in-3-ol an diesen Lindlar-Katalysatoren beschrieben.

Aus DE A 26 19 660 ist schließlich ein Verfahren zur Herstellung von Butendiol bekannt, bei dem Butindiol in einem inerten Lösungsmittel in Anwesenheit eines Katalysators, der mit Kohlenmonoxid behandeltes metallisches Pd enthält, hydriert wird.

Dieses Verfahren kann zusätzlich in Gegenwart von etwa 200 bis 2000 ppm CO im Hydrierwasserstoff durchgeführt werden.

Auch die Verwendung eines Pd/BaSO₄-Katalysators zur Herstellung von Butendiol ist aus der DE A 26 05 241 bekannt.

Aus M. Freifelder, "Practical Catalytic Hydrogenation", Wiley-Interscience, New York, 1971, Seiten 84 bis 126, ist eine Übersicht der technisch verwendeten Katalysatorsysteme für die Partialhydrierung von Dreifachbindungen zu olefinischen Doppelbindungen bekannt.

Alle genannten Verfahren haben den Nachteil, daß ein suspendierter Katalysator mit hohem Pd-Gehalt Verwendung findet. Der Katalysator muß nach erfolgter Hydrierung durch Absetzen und Filtrieren vom Reaktionsprodukt abgetrennt werden.

Es hat sich gezeigt, daß in technischem Maßstab die vollständige Abtrennung des pulverförmigen Katalysators nur mit sehr großem Aufwand möglich ist. Spuren von Katalysatorresten im Endprodukt verursachen aber bei der Weiterverarbeitung oder bei der sonstigen Verwendung der Alkene Schwierigkeiten. So hat es nicht an Versuchen gefehlt, einen Festbettkatalysator mit hoher Abriebfestigkeit für die partielle Hydrierung der Dreifachbindung in Alkinen in flüssiger Phase zu entwickeln.

In EP 0 841 314 wird ein Verfahren zur Hydrierung von 3,7,11,15-Tetramethyl-1-hexadecin-3-ol (Dehydroisophytol) beschrieben, bei dem an amorphen Metallegierungen, wie Pd₈₁Si₁₉ in überkritischem Kohlendioxid gearbeitet wird, die zur Steigerung der Selektivität bei der Hydrierung zum Isophytol mit Pb dotiert wurden. Zusätzlich mußte bei diesem Verfahren dem Hydriergemisch eine Schwefelverbindung, wie 1,2-Bis-(2-hydroxyethylthio)ethan, zugesetzt werden, um eine gute Ausbeute zu erreichen. Die aufwendige Katalysatorherstellung, die Abtrennung und Rückführung des Kohlendioxids und die zusätzliche Verwendung einer schwefelhaltigen Verbindung lassen das Verfahren als sehr aufwendig erscheinen.

Aus EP 0 412 415 ist ein Festbettkatalysator für die Hydrierung von 3,7-Dimethyl-oct-1-in-3-ol (Hydrodehydrolinalool) zu 3,7-Dimethyl-oct-1-en-3-ol (Hydrolinalool) bekannt, der als Aktivkomponente Palladium und als Inhibitor Metalle, wie Sn, Pb, Zn, Cd, Sb oder Bi enthält. Die in diesem Patent beschriebenen, mit Inhibitoren dotierten monolithischen Palladium-Festbettkatalysatoren ermöglichen es, die nachteilige Suspensionsfahrweise durch die technisch wesentlich vorteilhaftere Riesel- oder Sumpffahrweise am Festbettkatalysator zu ersetzen. Die recht hohe Abriebfestigkeit dieser Katalysatormonolithe ermöglicht eine sehr hohe Gas- und Flüssigkeitsbelastung. Leider hat sich bei der kontinuierlichen Durchführung des in diesem Patent beschriebenen Verfahrens an mit Bismut dotierten Palladium-Festbettkatalysatoren über längere Zeiträume gezeigt, daß die Selektivität der Hydrierung von Hydrodehydrolinalool zu Hydrolinalool langsam abnimmt, d.h. daß das Reaktionsprodukt ansteigende Mengen des vollständig hydrierten 3,7-Dimethyl-octan-3-ols enthält, was daran liegt, daß die Bismut-Dotierung verloren geht.

In EP 754 664 A wird beschrieben, daß das Verfahren gemäß EP B1 412 415 dadurch verbessert werden kann, daß man dem Hydriergas geringe Mengen an CO zudosiert. Nachteilig an diesem Verfahren ist, daß die Raum-Zeit-Ausbeuten noch nicht optimal sind und daß die Katalysatoren bei kontinuierlicher Fahrweise in technischem Maßstab nicht über ausreichend lange Zeiträume stabil und selektiv genug sind.

In den Verfahren gemäß EP 754 664, EP 0 841 314 und EP 412 415 wird nicht beachtet, daß sich bei der Hydrierung von Alkinen stets durch Oligomerisierung Hochsieder bilden, die die Gesamtselektivität negativ beeinflussen. Diese Hochsiederbildung ist aber aus der Literatur (vgl. G. Ertl et al. in "Handbook of Heterogeneous Catalysis", VCH, 1997, Seite 2172) bekannt und auch in EP 0 827 944 beschrieben.

In EP 827 944 wird ein Verfahren zur Hydrierung von mehrfach ungesättigten C₂-C₈-Kohlenwasserstoffen an den aus EP 412 415 bekannten Festbettkatalysatoren beschrieben, wobei die Dotierungskomponenten aus einer größeren Gruppe von Metallen ausgewählt werden und die Katalysatorherstellung um die Möglichkeit der Tränkung der Trägermaterialien erweitert wurde. Allerdings ist die Anwendung auf C₂-C₈-Kohlenwasserstoffe beschränkt. Aus der Literaturstelle G. Ertl et all. in -Handbook of Heterogeneous Catalysis", VCH, 1997, Seiten 2202-2204, ist aber bekannt, daß die Selektivität bei der Partialhydrierung von Alkinen stark von dem zu hydrierenden Alkin abhängt, da sich Faktoren wie Stoff-und Wärmetransport, Adsorption und Oberflächenreaktionen auf dem Katalysator stark auf die Selektivität auswirken. Gerade die Faktoren Stoff- und Wärmetransport hängen von der Viskosität des Reaktionsmediums ab (vgl. beispielsweise. M. Baerns, H. Hofmann, A. Renken in "Chemische Reaktionstechnik", Georg Thieme Verlag Stuttgart, 2. Auflage, 1992, Seiten 67-97), welche für Moleküle höheren Molekulargewichtes im allgemeinen hoch ist.

Die US-A-4,517,395 betrifft die Verfahren zur selektiven Hydrierung von Kohlenwasserstoffen mit mindestens 3 Kohlenstoffatomen und mehreren Doppel- oder Dreifachbindungen an geträgertan Palladiumkatalysatoren in Gegenwart von Wasserstoff und Kohlenmonoxid.

Die US-A-5,516,851 offenbart geträgerte Katalysatoren die herstellbar sind durch Lösen einer katalytisch aktiven Komponente in einem Lösungsmittel, Zugabe eines organischen Polymers, das bezüglich seines Eigengewichts mindestens die zehnfache Menge Wasser binden kann, Vermischen des Polymers mit einem Träger, und Schmelzen, Trocknen und Calzinieren.

Die US-A-4,490,481 betrifft Katalysatoren, die erhältlich sind durch: Vermischen einer Palladium-Verbindung mit einem anorganischen Träger, Rösten in Gegenwart eines Sauerstoff-haltigen Gutes. Behandlung mit einem Reduktionsmittel, Vermischen mit einem Goldhalogenid und abermaliger Behandlung mit einem Reduktionsmittel.

Es war daher die Aufgabe der Erfindung, ein Verfahren für die Herstellung von Alkenen höherer Molekulargewichte, d.h. Alkenen mit etwa 10 bis 30 C-Atomen, vorzugsweise von monosubstituierten Alkenen mit 10 bis 30 C-Atomen, durch Partialhydrierung der entsprechenden Alkine zu entwickeln, das die Nachteile der Suspensionsfahrweise nicht aufweist, technisch einfach umzusetzen ist und mit einfach herzustellenden und langzeitstabilen Katalysatoren arbeitet, die eine hohe Gesamtselektivität aufweist und möglichst wenig der überhydrierten Produkte und Hochsieder entstehen läßt.

Es wurde nun überraschenderweise gefunden, daß man monolithische Palladium-Festbett-Trägerkatalysatoren, die durch Tränken des getemperten Trägermaterials mit einer Palladiumsalzlösung erhalten wurden und in EP 0 827 944 bereits für die Partialhydrierung von niedermolekularen Alkinen beschrieben sind, mit guten Selektivitäten auch für C₁₀- bis C₃₀-Alkine verwenden kann, wenn man dem Hydrierwasserstoff Mengen an CO zusetzt, die im Bereich von 10 bis 2000 ppm liegen oder das zu hydrierende Alkin mit einer Verbindung mischt, die sich geringem Maße unter CO-Abspaltung zersetzt, ansonsten aber in die Hydrierung nicht weiter eingreift.

Gegenstand der Erfindung ist dementsprechend ein Verfahren zur Herstellung von Alkenen durch Partialhydrierung von Alkinen in der Flüssigphase bei Temperaturen von 20 bis 250°C und Wasserstoffpartialdrucken von 0,3 bis 200 bar an Palladium-Festbett-Trägerkatalysatoren, die durch Tempern des Trägermaterials an der Luft, Abkühlen, Aufbringen einer Palladiumverbindung und ggf. zusätzlich noch anderer Metallionen zu Dotierungszwecken, Verformen und Verarbeiten zu monolithischen Katalysatorelementen erhältlich sind, das dadurch gekennzeichnet ist, daß man
A) Alkine mit 10 bis 30 C-Atomen als Ausgangsverbindungen einsetzt,
B) die Palladiumverbindung und ggf. die anderen Metallionen durch Tränken des getemperten und abgekühlten Trägermaterials mit einer Palladiumsalze und ggf. andere Metallionen enthaltenden Lösung und anschließendes Trocknen auf das Trägermaterial aufbringt und
C) daß man dem Hydriergas 10 bis 2000 ppm Kohlenmomoxid (CO) zusetzt oder aber eine entsprechende Menge an CO in der flüssigen Phase durch geringfügige Zersetzung einer dem Reaktionsgemisch zugesetzten Verbindung, die unter den Reaktionsbedingungen CO abspaltet, entstehen läßt.

Besonders geeignet ist das Verfahren für die partielle Hydrierung von monosubstituierten Alkinen, wie 3,7-Dimethyl-oct-6-en-1-in-3-ol (Dehydrolinalool) oder 3,7-Dimethyl-oct-1-in-3-ol (Hydrodehydrolinalool). Die partielle Hydrierung von monosubstituierten Alkinen ist bekanntlich wesentlich problematischer als die von disubstituierten Alkinen, wie Butin-1,4-diol, da die monosubstituierten Alkine während der Hydrierung weiterreagieren können. Demgemäß ist mit dem erfindungsgemäßen Verfahren auch die Partialhydrierung von disubstituierten Alkinen, wie Butin-1,4-diol, möglich.

Als geeignete Ausgangsprodukte für das erfindungsgemäße Verfahren seien beispielsweise genannt:
monosubstituierte Alkine, wie Dehydrolinalool, Hydro-dehydrolinalool, 1-Ethinyl-2,6,6-trimethyl-cyclohexanol und 17-Ethinyl-androst-5-en-3β,17β-diol, 3.7,11.15-Tetramethyl-1-hexadecin-3-ol (Dehydroisophytol), 3,7,11-Trimethyldodec-1-in-3-ol, 3,7,11-Trimethyl-4-dodecen-1-in-3-ol und 3,7,11-Trimethyl-6-dodecen-1-in-3-ol (Dehydrodibydronerolidol) und clisubstituierte Alkine, wie 4-Methyl-4-hydroxy-2-decin, 1,1-Diethoxy-2-octin und Bis-(tetrahydro-2-pyranyloxy)-2-butin.

Die Ausgangsverbindungen können auch in Form einer Mischung aus zwei oder mehreren verschiedenen Alkinen eingesetzt werden. Aus der hierbei gebildeten Mischung aus verschiedenen Alkenen können dann die einzelnen Alkene in an sich bekannter Weise, beispielsweise durch Destillation abgetrennt werden.

Falls die Ausgangsalkine durch Umsetzung von Acetylen mit einem Keton hergestellt wurden, kann bei dem erfindungsgemäßen verfahren das unumgesetzte Keton in Mischung mit den Alkinen vorliegen. Dieses hat sogar den Vorteil, daß das Keton in der Lage ist, geringe Mengen CO abzuspalten, was eine Zudosierung von CO erübrigen kann.

Als Katalysator-Trägermaterial können Gewebe aus anorganischen Materialien, wie Al₂O₃ und/oder SiO₂ oder aber Gewebe aus Drähten aus Kunststoffen, wie Polyamiden, Polyestern, Polypropylen, Polytetrafluorethylen verwendet werden. Besonders geeignet sind jedoch folien- bzw. gewebeartige Metalltrager, d.h. Folien oder Drahtgewebe aus Metallen, wie Eisen, Federstahl, Kupfer, Messing, Aluminium, Neusilber, Nickel, Chromstahl oder Chromnickelstählen. Besonders bewährt haben sich Folien oder Gewebe aus Werkstoffen mit den Werkstoffnummern 1.4767, 1.4401 und 1.4301. Die Bezeichnung dieser Werkstoffe mit den genannten Werkstoffnummern folgt den Angaben der Werkstoffnummern in der "Stahleisenliste", herausgegeben vom Verein Deutscher Eisenhüttenleute; 8. Aufl., Seiten 87, 89 und 106; Verlag Stahleisen mbH, Düsseldorf 1990. Der Werkstoff der Werkstoffnummer 1.4767 ist auch unter dem Namen Kanthal bekannt. Diese metallischen Trägermaterialien werden durch oxidative Temperung, vorzugsweise an der Luft, bei Temperaturen von 600 bis 1100°C, vorzugsweise 700 bis 1000°C vorbehandelt.

Das Aufbringen des Palladiums erfolgt durch einfaches Tränken des Trägermaterials mit Pd-haltigen Lösungen, die durch Auflösen von Salzen anorganischer oder organischer Säuren des Palladiums, vorzugsweise von Nitraten, in einem Lösungsmittel, vorzugsweise Wasser, hergestellt wird. Die Metallsalzlösung kann zudem zusätzlich ein oder mehrere Promotorelemente enthalten, die aus den Gruppen II bis V und IB bis VIIIB des Periodensystems der Elemente stammen können. Besonders bevorzugt werden als Dotierungsmetalle Cu, Ag und Au verwendet. Nach der Tränkung erfolgt ein Trocknungsschritt, bei dem das Gewebe vorzugsweise bewegt wird. Ein Calcinierungsschritt schließt sich an.

Die Mengen an aufgebrachtem Pd können im Bereich von 5 bis 1000 mg/m² Gewebefläche, vorzugsweise im Bereich von 30 bis 500 mg/m² liegen. Die Mengen an den zusätzlichen Promotoren liegen im allgemeinen im Bereich von etwa 0,5 bis 800 mg/m² Gewebefläche, vorzugsweise aber im Bereich von 1 bis 500 mg/m².

Das so mit Palladium beschichtete Trägermaterial kann anschließend durch Tempern bei Temperaturen von 200 bis 800°C, vorzugsweise 300 bis 700°C, für 0,5 bis 2 Stunden formiert werden. Je nach Art der Palladiumbeschichtung kann dieser Temperschritt nach dem Beschichten aber auch entfallen. Die so mit Pd beschichteten und ggf. nachgetemperten Katalysatorfolien, Katalysatornetze oder Katalysatorgewebe werden dann zweckmäßigerweise für den Einbau in den Hydrierreaktor in an sich bekannter Weise zu Monolithen bzw. zu Formkörpern, wie z.B. Sulzerpackungen, verformt. Dadurch lassen sich die gewünschten guten Strömungsverhältnisse im Reaktor herstellen.

Nach der Reduktion des Katalysators mit Wasserstoff bei Temperaturen von 20 bis 250°C, vorzugsweise 70 bis 200°C, die man vorteilhaft im Reaktor durchführt, ist der Katalysator für die erfindungsgemäße Partialhydrierung einsatzbereit.

Mit Vorteil gelingt das erfindungsgemäße Verfahren wenn man die Partialhydrierung kontinuierlich in einem Rohrreaktor in Riesel-oder Sumpffahrweise mit Produktrückführung bei Querschnittsbelastungen von 20 bis 500 m³/m²*h, vorzugsweise 100 bis 300 m³/m²*h, durchführt.

Weiterhin ist es von großem Vorteil, wenn man ebenso wie das flüssige Produkt auch das Hydriergasgemisch aus Wasserstoff und CO mit ähnlichen Querschnittsbelastungen im Kreis fährt.

Die Geschwindigkeit der Wasserstoffaufnahme ist ein Maß für die Selektivität. Wird zu viel Wasserstoff pro Zeiteinheit umgesetzt, erhält man einen hohen Anteil überhydrierter Nebenprodukte, wird zu wenig Wasserstoff pro Zeiteinheit umgesetzt, erhält man einen hohen Anteil an oligomeren, hochsiedenden Nebenprodukten. Da die Geschwindigkeit der Wasserstoffaufnahme von der CO-Konzentration im Hydriergemisch abhängt, eröffnet das erfindungsgemäße Verfahren die sehr vorteilhafte Möglichkeit, die Selektivität mittels der CO-Dosierung einzustellen.

Mit besonderem Vorteil gelingt die Partialhydrierung in technischem Maßstab, wenn man sie in der Sumpffahrweise durchführt, und das Kreisgas mittels des Flüssigkeitsstromes und einer geeigneten Vorrichtung, wie einem Flüssigkeits-Gas-Verdichter, in feinster Verteilung in den Reaktor eindüst. Zusammen mit der Formgebung der Katalysatormonolithe und der beschriebenen Begasung des Reaktors erzielt man hohe Raum-Zeit-Ausbeuten durch optimale Quervermischung und gute Hydrodynamik an der Katalysatorgrenzfläche. Die Partialhydrierungen werden, je nach Substanz, bei Temperaturen von 20 bis 250°C, vorzugsweise 60 bis 200°C durchgeführt.

Mit Vorteil wird die Partialhydrierung kontinuierlich in einem oder mehreren hintereinandergeschalteten Reaktoren durchgeführt. Der Wasserstoffpartialdruck liegt im allgemeinen im Bereich von 0,3 bis 200 bar, vorzugsweise 0,5 bis 20 bar. Die Hydrierungen können mit und ohne Abgas gefahren werden.

Mit Hilfe des erfindungsgemäßen Verfahrens ist es möglich, zahlreiche als Riechstoffe oder Zwischenprodukte für Wirkstoffe, wie Vitamine, benötigte Alkene, insbesondere monosubstituierte Alkene, wie Linalool, Hydrolinalool, 3,7,11,15-Tetramethyl-1-hexadecen-3-ol (Isophytol), 3,7,11-Trimethyl-1-dodecen-3-ol (Tetrahydronerolidol), 3,7,11-Trimethyl-1,4-dodekadien-3-ol oder 3,7,11-Trimethyl-1,6-dodekadien-3-ol (Dihydronerolidol), auch in technischen Maßstab in einem kontinuierlichen Verfahren an relativ einfach herstellbaren, nur wenig Pd enthaltenden, abriebfesten und über lange Zeiträume stabilen Katalysatoren in guten Ausbeuten, guten Raum-Zeit-Ausbeuten und gleichbleibend guten Selektivitäten aus den entsprechenden Alkinen herzustellen.

Die Durchführung der Katalysatorherstellung und der erfindungsgemäßen Partialhydrierung im Vergleich mit derjenigen gemäß dem nächsten Stand der Technik wird mit den folgenden Beispielen näher erläutert.

### Beispiel 1 (Vergleichsbeispiel)

A. Katalysatorherstellung durch Bedampfen von Metallgewebe
   Ein glattes Kanthalgewebe (Werkstoffnummer 1.4767) mit einer Maschenweite von 180 µm und einem Drahtdurchmesser von 112 µm wurde für 5 Stunden (h) bei 950°C an der Luft getempert. Ein 20 cm breiter Gewebestreifen wurde auf die in einer Ultrahochvakuum-Bedampfungsanlage installierte Wickelvorrichtung aufgespannt und anschließend kontinuierlich bei einem Druck von 10⁻⁶ mbar mit 2 nm Pd bedampft. Durch Rückspulen des Gewebes wurde dieses in einem zweiten Bedampfungsschritt mit 0,7 nm Bi belegt. Nach dem Aufdampfen wurde das Katalysatorvorprodukt 30 Minuten (min) bei 600°C in einem Elektro-Muffelofen formiert. Dazu wurde der Temperofen in 40 Minuten (min) auf 600°C aufgeheizt, 30 min bei dieser Temperatur gehalten und dann abgeschaltet. Nach dem Erkalten wurde der Katalysator aus dem Muffelofen entnommen und zu einem Monolithen verformt.
B. Diskontinuierliche selektive Hydrierung von 3,7,11,15-Tetramethyl-1-hexadecin-3-ol (Dehydroisophytol) zu 3,7,11,15-Tetramethyl-1-hexadecen-3-ol (Isophytol) ohne Zufuhr von CO.
   Es wurde das gemäß Beispiel 1A hergestellte Pd/Bi-Trägerkatalysatormaterial in Form eines Metallmonolithen mit 13,2 mm Durchmesser und 200 mm Höhe in einen Rohrreaktor eingebracht. 300 g eines Gemisches aus Dehydroisophytol enthaltend ca. 18 Gew.-% 6,10,14-Trimethyl-pentadecan-2-on (Hexahydrofarnesylaceton) wurde in Sumpffahrweise unter Rückführung mit einer Querschnittsbelastung von 200 m³/m²*h über den Katalysator geleitet. Gleichzeitig mit dem Flüssigkeitsstrom wurde Wasserstoff bei einem Partialdruck von 2 bar im Kreis gefahren. Im Abgas, welches in der Zusammensetzung dem Kreisgas entsprach, wurde nach 60 min eine CO-Konzentration von 20 ppm gemessen, was aus dem Keton entstanden war, da kein CO zugefahren wurde. Bei einer Temperatur von 110°C wurde nach 170 min Vollumsatz erzielt. Die Gesamtselektivität betrug 93,2 %, wobei sich die Nebenprodukte auf überhydriertes Produkt und Rückstand im Verhältnis 1:2,8 verteilten.

### Beispiel 2

A. Katalysatorherstellung
   Das gleiche glatte Kanthalgewebe wie in Beispiel 1A wurde 5 h bei 900°C in Anwesenheit von Luft getempert. Ein 20 cm breiter Gewebestreifen wurde auf eine Wickelvorrichtung aufgespannt und anschließend kontinuierlich durch eine Tränkbad transportiert, das eine wässrige Metallsalzlösung aus Palladiumnitrat und Silbernitrat enthielt. Das danach getrocknete Gewebeband hatte eine Beschichtung von 73 mg Pd/m² und 18 mg Ag/m². Das Katalysatorvorprodukt wurde 3 h bei 300°C in einem Elektro-Muffelofen formiert. Anschließend wurde der Katalysator wie in Beispiel 1A beschrieben zu einem Monolithen verformt.
B. Diskontinuierliche selektive Hydrierung von Dehydroisophytol zu Isophytol ohne Zufuhr von CO.

Das gemäß Beispiel 2A hergestellten Pd/Ag-Trägerkatalysatormaterial wurde in Form eines Metallmonolithen mit 13,2 mm Durchmesser und 200 mm Höhe in einen Rohrreaktor eingebracht. 300 g eines Gemisches aus Dehydroisophytol enthaltend ca. 18 Gew.-% Hexahydrofarnesylaceton wurden in Sumpffahrweise unter Rückführung mit einer Querschnittsbelastung von 200 m³/m² *h über den Katalysator geleitet. Gleichzeitig mit dem Flüssigkeitsstrom wurde Wasserstoff bei einem Partialdruck von 2 bar im Kreis gefahren. Im Abgas, welches in der Zusammensetzung dem Kreisgas entsprach, wurde nach 60 min eine CO-Konzentration von 20 ppm gemessen, was aus dem Keton entstanden sein muß, da kein CO zugefahren wurde. Bei einer Temperatur von 110°C wurde nach 125 min Vollumsatz erzielt. Die Gesamtselektivität betrug 95,0 % der Theorie, wobei sich die Nebenprodukte auf überhydriertes Produkt und Rückstand im Verhältnis 1:0,54 verteilten.

### Beispiel 3

A. Katalysatorherstellung
   Das gleiche glatte Kanthalgewebe wie in Beispiel 1A wurde 5 h bei 900°C in Gegenwart von Luft getempert. Ein 20 cm breiter Gewebestreifen wurde auf eine Wickelvorrichtung aufgespannt und anschließend kontinuierlich durch ein Tränkbad transportiert, das eine wässrige Metallsalzlösung aus Palladiumnitrat und Silbernitrat enthielt. Das danach getrocknete Gewebeband hatte eine Beschichtung von 146 mg Pd/m² und 73 mg Ag/m². Anschließend wurde das Katalysatorvorprodukt, wie in Beispiel 2A beschrieben, formiert und zu einem Monolithen verformt.
B. Diskontinuierliche selektive Hydrierung von 3,7,11-Trimethyl-6-dodecen-1-in-3-ol (Dehydrodihydronerolidol) zu 3,7,11-Trimethyl-1,6-dodekadien-3-ol (Dihydronerolidol) ohne Zufuhr von CO.

Es wurde das gemäß Beispiel 3A hergestellte Pd/Ag-Katalysatormaterial in Form eines Metallmonolithen mit 13,2 mm Durchmesser und 200 mm Höhe in einen Rohrreaktor eingebracht. 300 g eines Gemisches aus Dehydrodihydronerolidol enthaltend ca. 36 Gew.-% 6,10-Dimethyl-5-undecaen-2-on (H-Geranylaceton) wurde in Sumpffahrweise unter Rückführung mit einer Querschnittsbelastung von 200 m³/m²*h über den Katalysator geleitet. Gleichzeitig mit dem Flüssigkeitsstrom wurde Wasserstoff bei einem Partialdruck von 2 bar im Kreis gefahren. Im Abgas, welches in der Zusammensetzung dem Kreisgas entsprach, wurde nach 60 min eine CO-Konzentration von 20 ppm gemessen, was aus dem Keton entstanden sein muß, da kein CO zugefahren wurde. Bei einer Temperatur von 110°C wurde nach 180 min Vollumsatz erzielt. Die Gesamtselektivität betrug 93,6 %, wobei sich die Nebenprodukte auf überhydriertes Produkt und Rückstand im Verhältnis 1:1,1 verteilten.

### Beispiel 4

A. Katalysatorherstellung
   Die Katalysatorherstellung erfolgte wie in Beispiel 2A, nur daß die aus dem Gewebe hergestellten Monolithe andere Maße besaßen.
B. Diskontinuierliche selektive Hydrierung von Dehydroisophytol, zu Isophytol ohne Zufuhr von CO.

20 Monolithe des gemäß Beispiel 4A hergestellten Pd/Ag-Katalysators mit 35 mm Durchmesser und 50 mm Höhe wurden in einen Rohrreaktor eingebracht. 2200 g eines Gemisches aus Dehydroisophytol enthaltend ca. 18 Gew.-% Hexahydrofarnesylaceton wurde in Sumpffahrweise unter Rückführung mit einer Querschnittsbelastung von 200 m³/m²*h über den Katalysator geleitet. Gleichzeitig mit dem Flüssigkeitsstrom wurde Wasserstoff bei einem Partialdruck von 2 bar im Kreis gefahren. Im Abgas, welches in der Zusammensetzung dem Kreisgas entsprach, wurde nach 45 min eine CO-Konzentration von 96 ppm gemessen, was aus dem Keton entstanden sein muß, da kein CO zugefahren wurde. Bei einer Temperatur von 98°C wurde nach 60 min Vollumsatz erzielt. Die Gesamtselektivität betrug 94 % der Theorie, wobei sich die Nebenprodukte auf überhydriertes Produkt und Rückstand im Verhältnis 1:0,24 verteilten.

### Beispiel 5

A. Katalysatorherstellung
   Die Katalysatorherstellung erfolgte wie in Beispiel 2A beschrieben, nur daß die aus dem Gewebe hergestellten Monolithe andere Maße besaßen.
B. Kontinuierliche selektive Hydrierung von Dehydrodihydronerolidol zu Dihydronerolidol.

Vier Monolithe des gemäß Beispiel 4A hergestellten Pd/Ag-Katalysators mit 35 mm Durchmesser und 200 mm Höhe sowie ein Monolith mit 35 mm Durchmesser und 100 mm Höhe wurden in einen Rohrreaktor eingebracht. Ein zweiter Rohrreaktor wurde mit 5 Monolithen vom Durchmesser 27 mm und 50 mm Höhe befüllt. Der erste Reaktor wurde in Sumpffahrweise unter Rückführung mit einer Flüssigkeitsquerschnittsbelastung von 200 m³/m²*h und einer Wasserstoffquerschnittsbelastung von 200 m³/m²*h bei einem Gesamtdruck von 7 bar betrieben. Dem Wasserstoff wurde soviel CO zudosiert, daß das Abgas, welches in der Zusammensetzung dem Kreisgas entspricht, eine CO-Konzentration von 1200 bis 1500 ppm enthielt. Die Temperatur im ersten Reaktor betrug 110°C. Die Zufuhrmenge an einem Gemisch aus Dehydrodihydronerolidol enthaltend ca. 18 Gew.-% 6,10-Dimethyl-undecan-2-on (H-Geranylaceton), entsprach der Entnahmemenge aus dem Kreislauf des ersten Reaktors, welche kontinuierlich dem zweiten Reaktor zugeführt wurde. Der zweite Reaktor wurde in Sumpffahrweise im geraden Durchgang bei einem Druck von 4 bis 5 bar, einer Temperatur von 70 bis 95°C und einer CO-Konzentration im Wasserstoff im Bereich von 1000 bis 1500 ppm betrieben. Nach einer Laufzeit von 318 h wurde im ersten Reaktor bei einem Umsatz von 95,4 % eine Gesamtselektivität von 95,4 % erzielt. Der Restumsatz wurde im zweiten Reaktor (Nachreaktor) realisiert. Die Gesamtselektivität lag danach bei 96,2 %, wobei sich die Nebenprodukte auf überhydriertes Produkt und Rückstand im Verhältnis 1:1,0 verteilten. Die Raum-Zeit-Ausbeute betrug 0,62 l/l_{Kat}*h.

Nach einer Laufzeit von 732 h wurde im ersten Reaktor bei einem Umsatz von 92,8 % eine Gesamtselektivität von 97,0 % erzielt. Der Restumsatz wurde im Nachreaktor realisiert. Die Gesamtselektivität lag danach bei 97,4 %, wobei sich die Nebenprodukte auf überhydriertes Produkt und Rückstand im Verhältnis 1:1,1 verteilten. Die Raum-Zeit-Ausbeute betrug 0,76 l/l_{Kat}*h. Daran ist erkennbar, daß der eingesetzte Katalysator über einen langen Zeitraum hinweg weder einer Desaktivierung noch einer Verschlechterung der Selektivität unterliegt.

### Beispiel 6

A. Katalysatorherstellung
   Die Katalysatorherstellung erfolgte wie in Beispiel 2A beschrieben, nur daß die aus dem Gewebe hergestellten Monolithe andere Maße besaßen.
B. Kontinuierliche selektive Hydrierung von 3,7-Dimethyloct-1-in-3-ol (Hydrodehydrolinalool) zu 3,7-Dimethyloct-1-en-3-ol (Linalool).

Es wurden 20 Monolithe des gemäß Beispiel 4A hergestellten Pd/Ag-Katalysators mit 35 mm Durchmesser und 50 mm Höhe in einen Rohrreaktor eingefüllt. Ein zweiter Rohrreaktor wurde mit 10 Monolithen vom Durchmesser 27 mm und 50 mm Höhe befüllt. Der erste Reaktor wurde in Sumpffahrweise unter Rückführung mit einer Flüssigkeitsquerschnittsbelastung von 200 m³/m²*h und einer Wasserstoffquerschnittsbelastung von 200 m³/m²*h bei einem Gesamtdruck von 7 bar betrieben. Dem Wasserstoff wurde soviel CO zudosiert, daß das Abgas, welches in der Zusammensetzung dem Kreisgas entsprach, eine CO-Konzentration von 1200 bis 1500 ppm enthielt. Die Temperatur im ersten Reaktor betrug 104°C. Die Zufuhrmenge an Hydriergemisch zum zweiten Reaktor wurde aus dem Kreislauf des ersten Reaktors entnommen. Der zweite Reaktor wurde in Sumpffahrweise im geraden Durchgang bei einem Druck von 4 bis 5 bar, einer Temperatur von 70°C und einer CO-Konzentration im Wasserstoff im Bereich von 200 bis 500 ppm betrieben. Nach einer Laufzeit von 22 h wurde im ersten Reaktor bei einem Umsatz von 95,4 % eine Gesamtselektivität von 95,3 % erzielt. Der Restumsatz wurde im Nachreaktor realisiert. Die Gesamtselektivität lag danach bei 94,8 %, wobei sich die Nebenprodukte auf überhydriertes Produkt und Rückstand im Verhältnis 1:0,93 verteilten. Die Raum-Zeit-Ausbeute betrug 1,01 l/l_{Kat}*h.

### Beispiel 7 (Vergleichsbeispiel zu Beispiel 6)

A. Katalysatorherstellung
   Die Katalysatorherstellung verlief analog zum Beispiel 1A, nur daß die aus dem Gewebe hergestellten Monolithe andere Maße besaßen.
B. Kontinuierliche selektive Hydrierung von Hydrodehydrolinalool zu Linalool.

Es wurden 20 Monolithe des gemäß Beispiel 1A hergestellten Pd/Bi-Katalysators mit 35 mm Durchmesser und 50 mm Höhe in einen Rohrreaktor eingebracht. Ein zweiter Rohrreaktor wurde mit 20 Monolithen vom Durchmesser 27 mm und 50 mm Höhe befüllt. Der erste Reaktor wurde in Sumpffahrweise unter Rückführung mit einer Flüssigkeitsquerschnittsbelastung von 200 m³/m²*h und einer Wasserstoffquerschnittsbelastung von 200 m³/m²*h bei einem Gesamtdruck von 6 bar betrieben. Dem Wasserstoff wurde soviel CO zudosiert, daß das Abgas, welches in der Zusammensetzung dem Kreisgas entspricht, eine CO-Konzentration von 100 bis 300 ppm enthielt. Die Temperatur im ersten Reaktor betrug 90°C. Die Zufuhrmenge an Hydriergemisch zum zweiten Reaktor wurde aus dem Kreislauf des ersten Reaktors entnommen. Der zweite Reaktor wurde in Sumpffahrweise im geraden Durchgang bei einem Druck von 4 bis 5 bar, einer Temperatur von 70°C und einer CO-Konzentration im Wasserstoff im Bereich von 50 bis 150 ppm betrieben. Nach einer Laufzeit von 100 h wurde im ersten Reaktor bei einem Umsatz von 87,9 % eine Gesamtselektivität von 90,6 % erzielt. Der Restumsatz wurde im Nachreaktor realisiert. Die Gesamtselektivität lag danach bei 90,4 %, wobei sich die Nebenprodukte auf überhydriertes Produkt und Rückstand im Verhältnis 1:1,6 verteilten. Die Raum-Zeit-Ausbeute betrug 0,36 l/l_{Kat}*h.

### Beispiel 8

A. Katalysatorherstellung
   Die Katalysatorherstellung erfolgte wie in Beispiel 2A beschrieben, nur daß die aus dem Gewebe hergestellten Monolithe andere Maße besaßen.
B. Kontinuierliche selektive Hydrierung von Dehydroisophytol zu Isophytol.

Vier Monolithe des gemäß Beispiel 8A hergestellten Pd/Ag-Katalysators mit 35 mm Durchmesser und 200 mm Höhe sowie ein Monolith mit 35 mm Durchmesser und 100 mm Höhe wurden in einen Rohrreaktor eingebracht. Ein zweiter Rohrreaktor wurde mit 5 Monolithen vom Durchmesser 27 mm und 50 mm Höhe befüllt. Der erste Reaktor wurde in Sumpffahrweise unter Rückführung mit einer Flüssigkeitsquerschnittsbelastung von 200 m³/m² *h und einer Wasserstoffquerschnittsbelastung von 200 m³/m² *h bei einem Gesamtdruck von 7 bar betrieben. Dem Wasserstoff wurde soviel CO zudosiert, daß das Abgas, welches in der Zusammensetzung dem Kreisgas entspricht, eine CO-Konzentration von 800-900 ppm enthielt. Die Temperatur im ersten Reaktor betrug 103°C. Die Zufuhrmenge an einem Gemisch aus Dehydroisophytol enthaltend ca. 12 Gew.-% 6,10,14-Trimethylpentadecan-2-on (Hexahydrofarnesylaceton), entsprach der Entnahmemenge aus dem Kreislauf des ersten Reaktors, welche kontinuierlich dem zweiten Reaktor zugeführt wurde. Der zweite Reaktor wurde in Sumpffahrweise im geraden Durchgang bei einem Druck von 4-5 bar einer Temperatur von 110°C und einer CO-Konzentration im Wasserstoff im Bereich von 100 ppm betrieben. Nach einer Laufzeit von 754 h wurde im ersten Reaktor bei einem Umsatz von 97,4% eine Gesamtselektivität von 96,7% erzielt. Der Restumsatz wurde im zweiten Reaktor (Nachreaktor) realisiert. Die Gesamtselektivität lag danach bei 96,7%, wobei sich die Nebenprodukte auf überhydriertes Produkt und Rückstand im Verhältnis 1:0,38 verteilten. Die Raum-Zeit-Ausbeute betrug 0,67 l/l_{Kat}*h.

### Beispiel 9

A. Katalysatorherstellung
   Das gleiche glatte Kanthalgewebe wie in Beispiel 1A wurde 5 h bei 900°C in Gegenwart von Luft getempert. Ein 20 cm breiter Gewebestreifen wurde auf eine Wickelvorrichtung aufgespannt und anschließend kontinuierlich durch ein Tränkbad transportiert, das eine wässrige Metallsalzlösung aus Palladiumnitrat und Silbernitrat enthielt. Das danach getrocknete Gewebeband hatte eine Beschichtung von 278 mg Pd/m² und 70 mg Ag/m². Anschließend wurde das Katalysatorvorprodukt, wie in Beispiel 2A beschrieben, formiert und zu einem Monolithen verformt.
B. Diskontinuierliche selektive Hydrierung von 3,7,11-Trimethyl-dodec-1-in-3-ol zu 3,7,11-Trimethyl-1-dodecen-3-ol (Tetrahydronerolidol) ohne Zufuhr von CO.

Es wurde das gemäß Beispiel 9A hergestellte Pd/Ag-Katalysatormaterial in Form eines Metallmonolithen mit 13,2 mm Durchmesser und 200 mm Höhe in einen Rohrreaktor eingebracht. 300 g eines Gemisches aus 3,7,11-Trimethyldodec-1-in-3-ol enthaltend ca. 2 Gew.-% 6,10-Dimethylundecan-2-on (TH-Geranylaceton) wurde in Sumpffahrweise unter Rückführung mit einer Querschnittsbelastung von 200 m³/m²*h über den Katalysator geleitet. Gleichzeitig mit dem Flüssigkeitsstrom wurde Wasserstoff bei einem Partialdruck von 2 bar im Kreis gefahren. Im Abgas, welches in der Zusammensetzung dem Kreisgas entsprach, wurde nach 60 min eine CO-Konzentration von 20 ppm gemessen, was aus dem Keton entstanden sein muß, da kein CO zugefahren wurde. Bei einer Temperatur von 110°C wurde nach 165 min Vollumsatz erzielt. Die Gesamtselektivität betrug 94,5%, wobei sich die Nebenprodukte auf überhydriertes Produkt und Rückstand im Verhältnis 1:0,47 verteilten.

## Patentansprüche

1. Verfahren zur Herstellung von Alkenen durch Partialhydrierung von Alkinen in der Flüssigphase bei Temperaturen von 20 bis 250°C und Wasserstoffpartialdrucken von 0,3 bis 200 bar an Palladium-Festbett-Trägerkatalysatoren, die durch Tempern des Trägermaterials an der Luft, Abkühlen, Aufbringen einer Palladiumverbindung und ggf. zusätzlich noch andere Metallionen zu Dotierungszwecken, Verformen und Verarbeiten zu monolithischen Katalysatorelementen erhältlich sind, **dadurch gekennzeichnet, daß** man
A) Alkine mit 10 bis 30 C-Atomen als Ausgangsverbindungen einsetzt,
B) die Palladiumverbindung und ggf. die anderen Metallionen durch Tränken des getemperten und abgekühlten Trägermaterials mit einer Palladiumsalze und ggf. andere Metallionen enthaltenden Lösung und anschließendes Trocknen auf das Trägermaterial aufbringt und
C) daß man dem Hydriergas 10 bis 2000 ppm Kohlenmomoxid (CO) zusetzt oder aber eine entsprechende Menge an CO in der flüssigen Phase durch geringfügige Zersetzung einer dem Reaktionsgemisch zugesetzten Verbindung, die unter den Reaktionsbedingungen CO abspaltet, entstehen läßt.

2. Verfahren gemäß Anspruch 1, **dadurch gekennzeichnet, daß** man als Palladium-Festbett-Trägerkatalysator einen Trägerkatalysator verwendet, der aus metallischem Trägermaterial in Form eines Metallgewebes oder einer Metallfolie hergestellt worden ist.

3. Verfahren gemäß Anspruch 1, **dadurch gekennzeichnet, daß** die Verbindung, die unter den Reaktionsbedingungen CO abspaltet, in dem Alkin in Mengen von 0 bis 80 Gew.-% enthalten ist.

4. Verfahren gemäß Anspruch 1, **dadurch gekennzeichnet, daß** man als Ausgangsverbindung Mischungen aus zwei oder mehreren verschiedenen Alkinen einsetzt und die einzelnen Alkene aus der gebildeten Mischung aus verschiedenen Alkenen in an sich bekannter Weise durch Destillation abtrennt.

5. Verfahren gemäß Anspruch 1, **dadurch gekennzeichnet, daß** man es zur Herstellung von 3,7,11,15-Tetramethyl-1-hexadecen-3-ol (Isophytol), 3,7,11-Trimethyl-1-dodecen-3-ol (Tetrahydronerolidol), 3,7,11-Trimethyl-1,4-dodekadien-3-ol oder 3,7,11-Trimethyl-1,6-dodekadien-3-ol (Dihydronerolidol) aus den entsprechenden Alkinen verwendet.

6. Verfahren gemäß Anspruch 3, **dadurch gekennzeichnet, daß** man es zur Herstellung von 3,7-Dimethyl-oct-1,6-dien-3-ol oder 3,7-Dimethyl-oct-1-en-3-ol aus den entsprechenden Alkinen verwendet.

7. Verfahren gemäß Anspruch 1, **dadurch gekennzeichnet, daß** man die Partialhydrierung in einem Rohrreaktor in Riesel- oder Sumpffahrweise mit Produktrückführung bei Querschnittsbelastungen von 20 bis 500 m³/m².h durchführt.

8. Verfahren gemäß Anspruch 7, **dadurch gekennzeichnet, daß** man das Hydriergasgemisch aus Wasserstoff und CO im Kreis fährt und die Wasserstoffaufnahme - und damit die Selektivität - mittels der CO-Dosierung regelt.

9. Verfahren gemäß Anspruch 7, **dadurch gekennzeichnet, daß** man die Partialhydrierung in der Sumpffahrweise durchführt und das Kreisgas mittels einer geeigneten Vorrichtung in feinster Verteilung in den Reaktor eindüst.

10. Verfahren gemäß Anspruch 1, **dadurch gekennzeichnet, daß** man die Partialhydrierung bei einem Wasserstoffpartialdruck von 0,5 bis 20 bar durchführt.

11. Verfahren gemäß Anspruch 1, **dadurch gekennzeichnet, daß** man die Partialhydrierung kontinuierlich in einem oder mehreren hintereinandergeschalteten Reaktoren durchführt.

12. Verfahren gemäß Anspruch 1, **dadurch gekennzeichnet, daß** man einen Festbett-Trägerkatalysator verwendet, der **dadurch** erhältlich ist, daß er nach dem Beschichten des Trägermaterials mit Palladium zur Formierung nachgetempert wird.

## Claims

1. A process for the preparation of alkenes by partial hydrogenation of alkynes in the liquid phase at from 20 to 250°C and hydrogen partial pressures of from 0.3 to 200 bar over fixed-bed supported palladium catalysts which are obtainable by heating the support material in the air, cooling, applying a palladium compound and, if appropriate, additionally other metal ions for doping purposes, molding and processing to give monolithic catalyst elements, wherein
A) alkynes of 10 to 30 carbon atoms are used as starting compounds,
B) the palladium compound and, if appropriate, the other metal ions are applied to the support material by impregnation of the heated and cooled support material with a solution comprising palladium salts and, if appropriate, other metal ions and subsequent drying, and
C) from 10 to 2000 ppm of carbon monoxide (CO) are added to the hydrogenation gas or a corresponding amount of CO is allowed to form in the liquid phase by slight decomposition of a compound which is added to the reaction mixture and eliminates CO under the reaction conditions.

2. The process according to claim 1, wherein the fixed-bed supported palladium catalyst used is a supported catalyst which has been prepared from metallic support material in the form of a metal woven fabric or a metal foil.

3. The process according to claim 1, wherein the compound which eliminates CO under the reaction conditions is contained in the alkyne in amounts of from 0 to 80% by weight.

4. The process according to claim 1, wherein mixtures of two or more different alkynes are used as the starting compound and the individual alkenes are separated from the resulting mixture of different alkenes by distillation in a manner known per se.

5. The process according to claim 1, which is used for the preparation of 3,7,11,15-tetramethyl-1-hexadecen-3-ol (isophytol), 3,7,11-trimethyl-1-dodecen-3-ol (tetrahydronerolidol), 3,7,11-trimethyl-1,4-dodecadien-3-ol or 3,7,11-trimethyl-1,6-dodecadien-3-ol (dihydronerolidol) from the corresponding alkynes.

6. The process according to claim 3, which is used for the preparation of 3,7-dimethyloct-1,6-dien-3-ol or 3,7-dimethyloct-1-en-3-ol from the corresponding alkynes.

7. The process according to claim 1, wherein the partial hydrogenation is carried out in a tube reactor by the trickle-bed or liquid phase procedure with product recycling at cross-sectional loadings of from 20 to 500 m³/m².h.

8. The process according to claim 7, wherein the hydrogenation gas mixture comprising hydrogen and CO is circulated and the hydrogen absorption, and hence the selectivity, are controlled by means of the CO metering.

9. The process according to claim 7, wherein the partial hydrogenation is carried out by the liquid phase procedure and the cycle gas is injected into the reactor by means of a suitable apparatus in very fine distribution.

10. The process according to claim 1, wherein the partial hydrogenation is carried out at a hydrogen partial pressure of from 0.5 to 20 bar.

11. The process according to claim 1, wherein the partial hydrogenation is carried out continuously in one or more reactors connected in series.

12. The process according to claim 1, wherein a fixed-bed supported catalyst is used which is obtainable by subsequently heating it for forming after coating of the support material with palladium.

## Revendications

1. Procédé de préparation d'alcènes par hydrogénation partielle d'alcynes en phase liquide à des températures de 20 à 250°C et à des pressions partielles d'hydrogène de 0,3 à 200 bars sur des catalyseurs de support à lit fixe de palladium qu'on obtient par mise à température du matériau de support à l'air, refroidissement, application d'un composé de palladium et le cas échéant en outre encore d'autres ions métal à des fins de dopage, mise en forme et traitement en éléments de catalyseur monolithiques, **caractérisé en ce que** :
A) on met en oeuvre des alcynes avec 10 à 30 atomes de carbone comme composés de départ,
B) on applique sur le matériau de support le composé de palladium et le cas échéant les autres ions métal par immersion du matériau de support mis à température et refroidi avec une solution contenant du sel de palladium et le cas échéant d'autres ions métal et séchage subséquent, et
C) on ajoute au gaz d'hydrogénation de 10 à 2000 ppm de monoxyde de carbone (CO) ou on laisse apparaître une quantité appropriée de CO dans la phase liquide par dégradation légère d'un composé ajouté au mélange réactionnel qui se dissocie dans les conditions réactionnelles.

2. Procédé selon la revendication 1, **caractérisé en ce qu'**on utilise comme catalyseur de support à lit fixe de palladium, un catalyseur de support fabriqué en matériau de support métallique sous forme d'un tissu métallique ou d'une feuille de métal.

3. Procédé selon la revendication 1, **caractérisé en ce que** le composé qui libère du CO dans les conditions réactionnelles, est contenu dans l'alcyne dans des quantités de 0 à 80% en poids.

4. Procédé selon la revendication 1, **caractérisé en ce qu'**on met en oeuvre comme composé de départ, des mélanges de deux alcynes différents, ou plus, et qu'on sépare par distillation les différents alcènes du mélange formé de différents alcènes d'une manière connue en soi.

5. Procédé selon la revendication 1, **caractérisé en ce qu'**on l'utilise pour la fabrication de 3,7,11,15-tétraméthyl-1-hexadécène-3-ol (Isophytol), de 3,7,11-triméthyl-1-dodécène-3-ol (tétrahydronérolidol), de 3,7,11-triméthyl-1,4-dodécadiène-3-ol ou de 3,7,11-triméthyl-1,6-dodécadiène-3-ol (dihydroérolidol) à partir des alcynes correspondants.

6. Procédé selon la revendication 3, **caractérisé en ce qu'**on l'utilise pour la fabrication de 3,7-diméthyl-oct-1,6-dién-3-ol ou de 3,7-diméthyl-oct-1-ène-3-ol à partir des alcynes correspondants.

7. Procédé selon la revendication 1, **caractérisé en ce qu'**on effectue l'hydrogénation partielle dans un réacteur tubulaire à écoulement ou à résidu avec un recyclage du produit à des charges transversales de 20 à 500 m³/m².h.

8. Procédé selon la revendication 7, **caractérisé en ce que** le mélange de gaz d'hydrogénation composé d'hydrogène et de CO effectue un cycle et que l'absorption d'hydrogène - et ainsi la sélectivité - se règle au moyen du dosage de CO.

9. Procédé selon la revendication 7, **caractérisé en ce qu'**on effectue l'hydrogénation partielle par résidu et qu'on injecte le gaz cyclique dans le réacteur sous forme finement divisée au moyen d'un dispositif approprié.

10. Procédé selon la revendication 1, **caractérisé en ce qu'**on effectue l'hydrogénation partielle à une pression partielle d'hydrogène de 0,5 à 20 bars.

11. Procédé selon la revendication 1, **caractérisé en ce qu'**on effectue l'hydrogénation partielle en continu dans un ou plusieurs réacteurs placés l'un derrière l'autre.

12. Procédé selon la revendication 1, **caractérisé en ce qu'**on utilise un catalyseur de support à lit fixe qu'on obtient en le mettant ultérieurement à température après le revêtement du matériau de support avec du palladium pour la mise en forme.
